# FASCICULE DE BREVET EUROPEEN

(11) **EP 3 013 310 B1**
(45) Date de publication et mention de la délivrance du brevet: **21.10.2020**
(21) Numéro de dépôt: 14735501.0
(22) Date de dépôt: 25.06.2014
(51) Int. Cl.: A61K 8/29, A61K 8/37, A61K 8/891, A61Q 19/00

(54) **COMPOSITION COSMÉTIQUE STABLE CONTENANT UN MONOGLYCÉRIDE, UN ESTER TARTRIQUE DE MONOGLYCÉRIDE ET UNE CHARGE ENROBÉE**
STABILE KOSMETISCHE ZUSAMMENSETZUNG MIT EINEM MONOGLYCERID, TATARISCHES ESTER AUS DEM MONOGLYCERID UND BESCHICHTETER FÜLLSTOFF
STABLE COSMETIC COMPOSITION CONTAINING A MONOGLYCERIDE, A TARTARIC ESTER OF MONOGLYCERIDE, AND A COATED FILLER

(30) Priorité: 28.06.2013 FR 1356283
(43) Date de publication de la demande: 04.05.2016
(73) Titulaire: L'OREAL, 75008 Paris (FR)
(72) Inventeur: CASSIN, Guillaume, 94240 l'Hay les Roses (FR); PORET FRISTOT, Sylvie, F-94150 Rungis (FR)
(74) Mandataire: Lavoix
(86) Numéro de dépôt international: PCT/EP2014/063445
(87) Numéro de publication internationale: WO 2014/207066

(56) Documents cités:
- DE-A1-102011 077 037
- US-A1- 2007 154 500
- DATABASE WPI Week 201338 Thomson Scientific, London, GB; AN 2013-H55529 XP002722764, & CN 102 845 540 A (BEIJING OUKAIMITE TECHNOLOGY CO LTD) 2 janvier 2013 (2013-01-02)

## Description

L'invention concerne une composition cosmétique stable spécialement adaptée à limiter ou réduire la brillance ou l'aspect brillant de la peau, en particulier dans des conditions climatiques tropicales.

L'aspect luisant que présente la peau dans des conditions de température et d'humidité relative importantes, caractéristiques des zones tropicales, est lié à un flux de sébum et de sueur au cours de la journée. Ce phénomène de luisance de la peau affecte donc aussi bien les populations se déclarant ou non « peau grasse ». Il se traduit aussi par une sensation de peau collante et sale au cours de la journée qui est particulièrement mal perçue par les individus vivant dans ces zones du monde.

A ce jour, l'utilisation de particules de charges minérales ou organiques pour matifier la peau est connue de l'homme de l'art. Malheureusement, ces particules n'ont pour effet que d'agir sur le sébum mais pas sur la transpiration. Les compositions contenant de telles charges minérales ou organiques ne permettent pas de réduire durablement la brillance de peau dans des conditions extrêmes (température et humidité relative importantes). On connait par ailleurs l'utilisation des sels d'aluminiums pour réduire la transpiration mais pour des raisons de confort évidentes leur utilisation sur le visage n'est pas envisageable. US2007/0154500 divulgue des compositions cosmétiques ayant un effet matifiant sur la peau, lesdites compostions comprenant des particules concaves, un agent dispersant, et des particules poreuses qui peuvent avoir une structure multi-couche.

Il existe donc un besoin pour des compositions cosmétiques, de préférence confortables et hydratantes, réduisant durablement la brillance de peau dans des conditions de température et d'humidité relative importantes.

L'invention a pour but de fournir des compositions, notamment cosmétiques, permettant de limiter ou réduire, de préférence durablement, la brillance ou l'aspect luisant d'une peau exposée à un climat tropical.

L'invention a notamment pour but de fournir une composition cosmétique aqueuse stable dans le temps permettant de résoudre les problèmes mentionnés ci-dessus.

Par ailleurs, l'invention a également pour but de fournir une composition cosmétique agréable et/ou confortable pour l'utilisateur, notamment lors de son ressenti à l'application sur la peau.

La présente invention a également pour but de fournir une composition, notamment cosmétique, présentant des propriétés hydratantes.

L'invention a pour but de résoudre l'ensemble de ces problèmes techniques de manière industrielle, fiable, reproductible, et à faible coût. L'invention concerne une composition telle que définie dans les revendications.

De façon surprenante, les inventeurs ont découvert qu'il était possible d'atteindre les objectifs précités grâce à une composition, notamment cosmétique, comprenant au moins un monoglycéride et au moins un ester tartrique de monoglycéride, et au moins un pigment enrobé et/ou au moins une charge minérale enrobée.

L'invention décrit encore l'utilisation d'une association d'au moins un monoglycéride et d'au moins un ester tatrique diacétyl de monoglycéride pour limiter l'aspect luisant ou la brillance de la peau, notamment sous un climat tropical.

Au sens de l'invention, on entend par « climat tropical » tout spécialement un climat dans une zone du globe terrestre comprise entre les tropiques, généralement jusqu'à environ 15° ou 20° de latitude nord et sud. Il s'agit d'un climat non aride où la température moyenne mensuelle ne descend pas en dessous de 18°C tout au long de l'année. Les climats tropicaux présentent une saison sèche et une saison humide. En général, les climats tropicaux présentent des précipitations généralement comprises entre 1 et 2 m annuelles. L'invention couvre en particulier des compositions, notamment cosmétiques, adaptées au climat de la Chine, de l'Inde, du Brésil, et des pays aux conditions climatiques équivalentes.

Un climat tropical comprend donc des conditions de température et d'humidités relatives importantes enclines à générer un flux de sébum et de sueur importants au cours de la journée, même pour des peaux qui ne sont en dehors de ces climats pas particulièrement grasses. Ce phénomène de luisance ou brillance de la peau affecte donc aussi bien les populations se déclarant ou non « peaux grasses ».

Toutefois les compositions de l'invention ne sont pas limitées à leur utilisation sous un climat tropical et peuvent être utilisé sous tout type de climat, en particulier à fin de limiter l'aspect luisant ou brillant de la peau

Avantageusement, la composition comprend une phase aqueuse. Une composition selon l'invention peut comprendre plus de 50%, et de préférence entre 60% et 95%, et encore de préférence entre 65% et 95%, en masse de phase aqueuse par rapport à la masse totale de la composition.

La composition selon l'invention contient une phase lipogel.

Les systèmes aqueux basés sur l'association de monoglycéride et d'un ester tartrique de monoglycérides, sont communément appelés lipogels et sont utilisés en alimentaires depuis quelques années pour faire des margarines allégées. Un lipogel comprend généralement une matrice tridimensionnelle renfermant une phase continue non-aqueuse. Un lipogel est un systéme complexe généralement constitué de cristaux de corps gras percolés dans une phase aqueuse. La taille de tels cristaux est généralement de quelques dizaines de micromètres. Un lipogel est formé en général par refroidissement d'une composition formant à chaud (ici, température supérieure à 55°C) une phase lamellaire visible sous microscope optique en lumière bi-refringente. Une telle architecture tridimensionnelle peut contenir jusqu'à 98% d'une phase aqueuse.

Ces systèmes ont fait l'objet d'études structurales importantes. On peut citer en autres les articles suivants.
* Liquid Crystalline Phases in the Structuring of Food Products, 1998, Lebensmittel-Wissenschaft und-technologie, 31, 387-396,
* Investigation of the Gel to Coagel Phase Transition in Monoglycéride-Water Systems (Langmuir 1998, 14, 5757-5763)
* Lipid organization and dynamics of the monostearoylglycerol-water system. A 2H NMR study (Chemistry and Physics of lipids 109 (2001) 15-28)
* Rheological Characterization, Crystallization, and Gelation (Behavior of Monoglycéride Gels (Journal of Colloid and Interface Science 249, (2002) 412-422).

L'utilisation de stéarate de monoglycérides en cosmétique pour stabiliser des émulsions est connue depuis longtemps et peut être considérée comme dans le domaine public. Par contre, l'utilisation d'un ester tartrique de monoglycéride est, elle, moins courante.

Les compositions selon l'invention se présentent de préférence sous la forme de crèmes, de mousses, ou de sticks. Il est important de remarquer que les compositions selon l'invention si elles ont l'aspect de crèmes cosmétiques ont, quand on les observe au microscope, une structure différente des émulsions classiques caractérisées par exemple par un tapis régulier de gouttelettes. Les compositions selon l'invention se caractérisent notamment par un aspect fortement biréfringent en lumière polarisée typique de leur nature semi cristalline.

Les pourcentages des constituants sont exprimés en masse par rapport à la masse totale de la composition, sauf indication contraire.

### Monoglycéride

Un monoglycéride, ou monoacylglycérol (MAG), est un glycéride formé d'un résidu d'acide gras combiné à un résidu de glycérol par une liaison ester. On peut les classer en deux groupes, les 1-monoacyglycérols et les 2-monoacylglycérols selon que le groupe acyle est en position 1 ou 2 du résidu glycérol.

Selon un mode de réalisation particulier, la composition comprend à titre de monoglycéride un ou plusieurs monoglycérides comprenant une chaine alkyl, saturée ou insaturée, comprenant de 12 à 22 atomes de carbone.

De préférence, le monoglycéride comprend une chaine alkyl, saturée ou insaturée, comprenant 16 ou 18 atomes de carbone.

Dans le cas présent, on s'intéresse tout particulièrement au 1-monoacylglycérols, comme ceux de formule suivante (exemple en C18) :

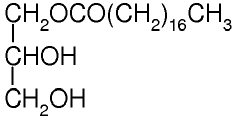

La longueur de la chaine d'acide gras peut aller de C12 à C22 préférentiellement on choisit les esters de monoglycérides en C16 ou C18.

La matière première utilisée est importante en ce sens que les monoglycérides utilisés permettent de former une phase lipogel. En particulier on utilise des monoglycérides contenant moins de 10% de di-glycérides résiduels. On utilise de préférence des stéarates de monoglycérides, par exemple commercialisés sous la dénomination DIMODAN HP par la société DANISCO, ou ceux commercialisés sous la dénomination TEGIN 90 par la société EVONIK GOLDSCHMIDT.

La teneur en monoglycérides des formulations selon l'invention va de 1% à 20%, préférentiellement de 2 à 10% et très préférentiellement de 3 à 8% en masse par rapport à la masse totale de ladite composition.

### Ester tartrique de monoglycérides d'acides gras

Les esters tartriques de monoglycérides d'acides gras sont généralement obtenus en greffant un résidu tartrique en position 3 du résidu glycérol d'un 1-monoacylglycérol. La longueur de la chaine d'acide gras peut aller de C12 à C22, préférentiellement on choisira les esters de monoglycérides en C16 ou C18.

L'ester tartrique de monoglycéride est de préférence un ester tartrique diacétyl de monoglycéride comprenant une chaine alkyl, saturée ou insaturée, comprenant de 12 à 22 atomes de carbone, et de préférence, comprenant 16 ou 18 atomes de carbone.

Dans le cas présent, on s'intéresse tout particulièrement aux esters diacétyl tartriques de monoglycérides en C18, comme ceux de formule suivante :

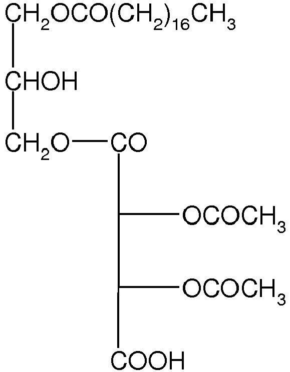

L'invention couvre les différents isomères de l'acide tartrique et leurs mélanges, y compris les mélanges racémiques.

Ces ingrédients et leurs utilisations sont décrits pages 88 à 95 de la brochure Functional Ungredients for Food éditée par DANISCO et accessible sur le site http://www.danisco.com/.

On peut choisir ces esters parmi un ester tartrique de mono- et diglycérides d'acides gras (additif E472d), et un ester monoacétyltartrique de mono- et diglycérides d'acides gras (additif E472e). On recherche principalement des esters de monoglycérides et de diglycérides avec une pureté de l'ordre de 80% et plus.

La teneur en ester tartrique de monoglycéride des formulations selon l'invention va de 0,05% à 5%, préférentiellement de 0,1 à 2% et très préférentiellement de 0,1 à 1 % en masse par rapport à la masse totale de ladite composition. Typiquement on utilise une teneur en ester tartrique de monoglycéride de 0,2-0,5% en masse par rapport à la masse totale de ladite composition.

### Charges minérales et pigments enrobés

Les pigments et/ou charges minérales utilisés selon l'invention sont traités en surface, totalement ou partiellement, par au moins un agent de traitement hydrophobe.

Au sens de l'invention, le traitement de surface d'un pigment et/ou charge minérale désigne de manière générale le traitement en surface total ou partiel par un agent de surface, absorbé, adsorbé, ou greffé sur ledit pigment et/ou charge minérale. Les pigments et/ou charges minérales traités en surface peuvent être préparés selon des techniques de traitement de surface de nature chimique, électronique, mécano-chimique ou mécanique bien connues de l'homme de l'art. On peut également utiliser des produits commerciaux. L'agent de surface peut être absorbé, adsorbé ou greffé sur les pigments et/ou charges minérales par évaporation de solvant, réaction chimique et/ou création d'une liaison covalente.

Par « pigment » on entend désigner une particule solide, blanche ou colorée, naturellement insoluble dans les phases hydrophiles et lipophiles liquides usuellement employées en cosmétique ou rendue insoluble par formulation sous forme de laque, le cas échéant. On peut citer comme pigment, les pigments organiques et inorganiques tels que ceux définis et décrits dans Ullmann's Encyclopedia of Industrial Chemistry " Pigment organics ", 2005 Wiley-VCH Verlag GmbH & Co. KGaA, Weinheim, et "Pigments, Inorganic, 1. General" 2009 Wiley-VCH Verlag GmbH & Co. KGaA, Weinheiml.

Les charges minérales selon l'invention sont des pigments minéraux.

On peut citer les pigments azoiques qui contiennent un ou plusieurs groupes azoiques A-N=N-B avec A représentant un (hétéro)aryle éventuellement substitué, B représentant (hétéro)aryle éventuellement substitués ou -CH[C(0)-R]-C(0)-X,-A', A' représentant un (hétéro)aryle éventuellement substitué et R représentant un atome d'hydrogène ou un groupe (C,-C6)alkyle, avec les groupes A, A' et B (hétéro)aryles qui ne contiennent pas de groupe solubilisants tels que -SO3H, -COOH. Ils peuvent être particulièrement monoazoiques dont les 3-Naphthols, les pigments monoazopyrrolones, les pigments Benzimidazolone ; les pigments diazoiques tels que les pigments diazodiarylides et les Bis(N-acétoacétarylide), les pigments triazoiques ou tétraazoiques. On peut également citer les Pigments Azoïques à Complexes Metalliques ou « azo metal complex pigment ».

D'autres pigments sont également intéressants, il s'agit des pigments Isoindolinones et Isoindolines, les pigments Phthalocyanine ; les pigments Quinacridone ; les pigments Perinones ; les pigments Perylène ; les pigments Anthraquinones telles que les Pigments Hydroxyanthraquinones ; les pigments Aminoanthraquinones dont les Acylaminoanthraquinones et les pigments Anthraquinones Azoïques ; les Anthraquinones Heterocycliques ; les pigments polycarbocycliques Anthraquinones, les Pigments Pyranthrones ; les pigments Anthanthrones ; les pigments Dicétopyrrolopyrroles (DPP) ; les pigments Thioindigo ; les pigments Dioxazines ; les pigments Triphénylmethanes ; les pigments Quinophthalones ; et les pigments Fluorescents.

Dans le cadre de la présente invention, le pigment peut être au moins en partie organique. Selon un mode de réalisation de l'invention, le pigment est un pigment organique. Selon un autre mode de réalisation de l'invention, le pigment est un pigment minéral.

A titre illustratif des pigments utilisables dans la présente invention, on peut citer le noir de carbone, l'oxyde de titane, l'oxyde de chrome, les pigments de type D&C, FD&C et leurs laques, et notamment ceux connus sous les dénominations D&C Blue n°4, D&C Brown n°1, FD&C Green n°3, D&C Green n°5, D&C Green n°6, FD&C Green n°8, 20 D&C Orange n°4, D&C Orange n°5, D&C Orange n°10, D&C Orange n°11, FD&C Red n°4, D&C Red n°6, D&C Red n°7, D&C Red n°17, D&C Red n°21, D&C Red n°22, D&C Red n°27, D&C Red n°28, D&C Red n°30, D&C Red n°31, D&C Red n°33, D&C Red n°34, D&C Red n°36, FD&C Red n°40, FD&C Red 40 lake, D&C Violet n°2, Ext. D&C Violet n°2, FD & C Blue n°1, D&C Yellow n°6, FD&C Yellow n°6, D&C Yellow n°7, 25 Ext. D&C Yellow n°7, D&C Yellow n°8, D&C Yellow n°10 ou D&C Yellow n°11, étant entendu que lorsque ledit pigment n'est pas naturellement insoluble dans les phases hydrophiles et lipophiles usuellement employées en cosmétique, il est utilisé sous forme d'une laque correspondante. A titre d'exemple de laques, on peut notamment citer les laques à base de baryum, strontium, calcium, aluminium, ou encore les diceto pyrrolopyrrole. Comme autres exemples de pigments utilisables dans la présente invention, on peut notamment citer les pigments minéraux traités en surface et/ou enrobés, et notamment le dioxyde de titane, les oxydes de zirconium ou de cérium, ainsi que les oxydes de zinc, de fer (noir, jaune ou rouge) ou de chrome, le violet de manganèse, le bleu outremer, l'hydrate de chrome et le bleu ferrique, ou encore les poudres métalliques comme la poudre d'aluminium, la poudre de cuivre, la poudre d'or et la poudre d'argent. On peut également citer les pigments a effet optique tels que les particules comportant un substrat organique ou minéral, naturel ou synthétique, par exemple le verre, les résines acrylique, le polyester, le polyuréthane, le polyéthylène téréphtalate, les céramiques ou les alumines, ledit substrat étant recouvert ou non de substances métalliques comme l'aluminium, l'or, l'argent, le platine, le cuivre, le bronze, ou d'oxydes métalliques comme le dioxyde de titane, l'oxyde de fer, ou l'oxyde de chrome. Il peut également s'agir de nacres. Par nacres, il faut comprendre des pigments irises, notamment produits par certains mollusques dans leur coquille ou bien synthétises. Les pigments nacrés peuvent être choisis parmi le mica recouvert de titane ou d'oxychlorure de bismuth, le mica titane recouvert avec des oxydes de fer, le mica titane recouvert avec notamment du bleu ferrique ou de l'oxyde de chrome, le mica titane recouvert avec un pigment organique du type précité ainsi que les pigments nacres à base d'oxychlorure de bismuth. On peut également utiliser des pigments interférentiels, notamment à cristaux liquides ou multicouches. Il peut également s'agir de pigments ayant une structure qui peut être par exemple de type sericite/oxyde de fer brun/dioxyde de titane/silice. Il peut également s'agir de pigments ayant une structure qui peut être par exemple de type microsphères de silice contenant de l'oxyde de fer.

A titre d'exemples de pigments convenant tout particulièrement à la mise en œuvre de la présente invention, on peut notamment citer le D&C Red n°7, l'oxyde de titane, l'oxyde de chrome, les laques des pigments de type D&C et FD&C cites ci-dessus, et notamment le D&C Red n°22 lake, le Yellow n°6 lake, le FD&C Blue n°1 lake. Les pigments conformes à l'invention peuvent se présenter sous forme de poudre ou de pâte pigmentaire. Les pigments conformes à l'invention peuvent par exemple être choisis parmi les pigments blancs ou colorés, les laques, les pigments à effets spéciaux tels que les nacres ou les paillettes, et leurs mélanges. A titre d'exemples de pigments minéraux blancs ou colorés, on peut citer les oxydes de zirconium ou de cérium, les oxydes de chrome, le violet de manganèse, le bleu outremer, l'hydrate de chrome et le bleu ferrique. A titres d'exemples de pigments organiques blancs ou colorés, on peut citer les composés nitroso, nitro, azo, xanthène, quinoléine, anthraquinone, phtalocyanine, de type complexe métallique, isoindolinone, isoindoline, quinacridone, périnone, pérylène, dicétopyrrolopyrrole, thioindigo, dioxazine, triphénylméthane, quinophtalone.

En particulier, les pigments organiques blancs ou colorés peuvent être choisis parmi le carmin, le noir de carbone, le noir d'aniline, le jaune azo, la quinacridone, le bleu de phtalocyanine, le rouge sorgho, les pigments bleus codifiés dans le Color Index sous les références CI 42090, 69800, 69825, 73000, 74100, 74160, les pigments jaunes codifiés dans le Color Index sous les références CI 11680, 11710, 15985, 19140, 20040, 21100, 21108, 47000, 47005, les pigments verts codifiés dans le Color Index sous les références CI 61565, 61570, 74260, les pigments oranges codifiés dans le Color Index sous les réfénces CI 11725, 15510, 45370, 71105, les pigments rouges codifiés dans le Color Index sous les références CI 12085, 12120, 12370, 12420, 12490, 14700, 15525, 15580, 15620, 15630, 15800, 15850, 15865, 15880, 17200, 26100, 45380, 45410, 58000, 73360, 73915, 75470, les pigments obtenus par polymérisation oxydante de dérivés indoliques, phénoliques tels qu'ils sont décrits dans le brevet FR 2 679 771.

On peut utiliser des pâtes pigmentaires de pigment organique telles que les produits vendus par la société HOECHST sous le nom : - JAUNE COSMENYL 10G : Pigment YELLOW 3 (CI 11710) ; - JAUNE COSMENYL G : Pigment YELLOW 1 (CI 11680) ; - ORANGE COSMENYL GR : Pigment ORANGE 43 (CI 71105) ; - ROUGE COSMENYL R : Pigment RED 4 (CI 12085) ; - CARMIN COSMENYL FB : Pigment RED 5 (CI 12490) ; - VIOLET COSMENYL RL : Pigment VIOLET 23 (CI 51319) ; - BLEU COSMENYL A2R : Pigment BLUE 15.1 (CI 74160) ; - VERT COSMENYL GG : Pigment GREEN 7 (CI 74260) ; - NOIR COSMENYL R : Pigment BLACK 7 (CI 77266). Les pigments conformes à l'invention peuvent aussi être sous forme de pigments composites tels qu'ils sont décrits dans le brevet EP 1 184 426. Ces pigments composites peuvent être composés notamment de particules comportant : - un noyau inorganique, - au moins un liant assurant la fixation des pigments organiques sur le noyau, et - au moins un pigment organique recouvrant au moins partiellement le noyau. Par laque, on entend les colorants adsorbés sur des particules insolubles, l'ensemble ainsi obtenu restant insoluble lors de l'utilisation. Les substrats inorganiques sur lesquels sont adsorbés les colorants sont par exemple l'alumine, la silice, le borosilicate de calcium et de sodium ou le borosilicate de calcium et d'aluminium, et l'aluminium. Parmi les colorants organiques, on peut citer le carmin de cochenille. A titre d'exemples de laques, on peut citer les produits connus sous les dénominations suivantes : D & C Red 21 (CI 45 380), D & C Orange 5 (CI 45 370), D & C Red 27 (CI 45 410), D & C Orange 10 (CI 45 425), D & C Red 3 (CI 45 430), D & C Red 7 (CI 15 850:1), D & C Red 4 (CI 15 510), D & C Red 33 (CI 17 200), D & C Yellow 5 (CI 19 140), D & C Yellow 6 (CI 15 985), D & C Green (CI 61 570), D & C Yellow 10 (CI 77 002), D & C Green 3 (CI 42 053), D & C Blue 1 (CI 42 090). Par pigments à effets spéciaux, on entend les pigments qui créent d'une manière générale une apparence colorée (caractérisée par une certaine nuance, une certaine vivacité et une certaine clarté) non uniforme et changeante en fonction des conditions d'observation (lumière, température, angles d'observation...). Ils s'opposent par-là même aux pigments blancs ou colorés qui procurent une teinte uniforme opaque, semitransparente ou transparente classique.

A titre d'exemples de pigments à effets spéciaux, on peut citer les pigments nacrés blancs tels que le mica recouvert de titane, ou d'oxychlorure de bismuth, les pigments nacrés colorés tels que le mica recouvert de titane et d'oxydes de fer, le mica recouvert de titane et notamment de bleu ferrique ou d'oxyde de chrome, le mica recouvert de titane et d'un pigment organique tel que défini précédemment ainsi que les pigments nacrés à base d'oxychlorure de bismuth. On peut également citer les pigments à effet interférentiel non fixés sur un substrat comme les cristaux liquides (Helicones HC de Wacker), les paillettes holographiques interférentielles (Geometric Pigments ou Spectra f/x de Spectratek). Les pigments à effets spéciaux comprennent aussi les pigments fluorescents, que ce soit les substances fluorescentes à la lumière du jour ou qui produisent une fluorescence ultraviolette, les pigments phosphorescents, les pigments photochromiques, les pigments thermochromiques et les quantum dots, commercialisés par exemple par la société Quantum Dots Corporation. Les quantum dots sont des nanoparticules semi conductrices luminescentes capables d'émettre, sous excitation lumineuse, un rayonnement présentant une longueur d'onde comprise entre 400 nm et 700 nm. Ces nanoparticules sont connues de la littérature. En particulier, elles peuvent être fabriquées selon les procédés décrits par exemple dans le US 6 225 198 ou US 5 990 479, dans les publications qui y sont citées, ainsi que dans les publications suivantes : Dabboussi B.O. et al "(CdSe)ZnS core-shell quantum dots : synthesis and characterisation of a size series of highly luminescent nanocristallites" Journal of physical chemistry B, vol 101, 1997, pp 9463-9475. et Peng, Xiaogang et al, "Epitaxial Growth of highly Luminescent CdSe/CdS core/shell nanocrystals with photostability and electronic accessibility" Journal of the American Chemical Society, vol 119, N°30, pp 7019-7029.

Les pigments conformes à l'invention sont de préférence des pigments colorés.

La variété des pigments mis en jeu permet d'obtenir une riche palette de couleurs, ainsi que des effets optiques particuliers tels que des effets métalliques, interférentiels. La taille d'un pigment autre que les nacres en solution est généralement comprise entre 10 nm et 10 pm, de préférence entre 50 nm et 5 pm, et encore plus préférentiellement entre 100 nm et 3 pm. La taille d'une nacre en solution est généralement comprise entre 1 et 200 pm, de préférence entre 1 et 80 pm, et encore plus préférentiellement entre 1 et 50 pm. Parmi les pigments minéraux, on peut citer à titre d'exemple le dioxyde de titane (rutile ou anastase) éventuellement traité en surface et codifié dans le Color Index sous la référence C177891 ; les oxydes de fer noir, jaune rouge et brun, codifiés sous les références C177499, 77492, 77491 ; le violet de manganèse (C177742) ; le bleu outremer (C177007) ; l'oxyde de chrome hydraté (C177289) ; le bleu ferrique (C177510).

Parmi les pigments organiques, on peut citer à titre d'exemple, le pigment YELLOW 3 vendu notamment sous la dénomination commerciale "JAUNE COVANOR W 1603 par la société WACKHERR (CI 17710), le "D & C RED n° 19" (CI 45170), le D & C RED n°9 (CI 15585), le D & C RED n°21 (CI 45380), le D & C ORANGE n°4 (CI 15510), le D & C ORANGE n° 5 (CI 45370), le D & C RED n° 27 (C145410), le D & C RED n° 13 (CI 15630), le D & C RED n° 7 (CI 15850-1), le D & C RED n° 6 (CI 15850-2), le D & C YELLOW n°5 (CI 19140), le D & C RED n°36 (CI 12085), le D & C ORANGE n° 10 (CI 45425), le D & C YELLOW n° 6 (CI 15985), le D & C RED n°30 (CI 73360), le D & C RED n° 3 (CI 45430), le noir de carbone (CI 77266), et les laques à base de carmin de cochenille (CI 75470).

On peut également utiliser des pigments nacrés qui peuvent être notamment choisis parmi les pigments nacrés blancs tels que le mica recouvert d'oxyde de titane, l'oxyde de bismuth ; les pigments nacrés colorés tels que le mica titane avec des oxydes de fer, le mica titane avec du bleu ferrique ou de l'oxyde de chrome, le mica titane avec un pigment organique de type précipité, ainsi que ceux à base d'oxychlorure de bismuth.

La teneur en pigments et/ou charges minérales enrobés peut avantageusement aller de 0,1% à 4% en masse, de préférence de 0,2% à 3% en masse par rapport à la masse totale de ladite composition.

### Agent de traitement hydrophobe (ou lipophile)

Selon un mode de réalisation particulier de l'invention, les pigments et/ou charges minérales peuvent être traités en surface ou enrobés par au moins un agent de traitement hydrophobe ou lipophile choisi parmi les agents de surface siliconés ; les agents de surface fluorés ; les agents de surface fluoro-siliconés ; les savons métalliques, les acides aminés N-acylés ou leurs sels tels que le disodium stearoyl glutamate ; la lécithine et ses dérivés ; le trisostéaryle titanate d'isopropyle ; le sébaçate d'isostéaryle ; les cires naturelles végétales ou animales, les cires synthétiques polaires; les esters gras ; les alcools gras ; les phospholipides, et leurs mélanges. Comme agents de surface siliconé on peut citer les polydiméthylsiloxanes.

L'agent de traitement peut représenter de 0,1 à 50% en masse, et en particulier de 0,5 à 5% en masse, de la masse totale du pigment enrobé ou de la charge minérale enrobée.

### Agent séquestrant

Dans un mode alternatif de réalisation de l'invention, les formules peuvent contenir en outre un ou plusieurs agents séquestrant.

Les agents séquestrant sont des molécules qui forment des complexes chimiques avec les ions métalliques, tels que le cuivre, le fer et le nickel.

De manière générale les agents séquestrants ou agents chélatants sont décrits dans l'Encyclopédie KIRK-OTHMER Encyclopedia of Chemical Technology John Wiley and sons Vol 5 pages 708 à 739.

Parmi les agents séquestrants utilisables dans la composition de l'invention, on peut citer par exemple l'acide éthylène diamine tétracétique (EDTA) et ses sels tels que le sel disodique d'éthylène diamine tétracétique (Disodium EDTA) ; les dérivés phosphoniques tels que l'acide hexaméthylène diamine tétra(méthylène phosphonique), l'acide éthylène diamine tétra(méthylène phosphonique), l'acide 1-hydroxyéthylidène 1,1-diphosphonique, l'acide aminotri(méthylène phosphonique), l'acide diéthylène-triamine penta(méthylène phosphonique), et leurs sels et notamment leurs sels de sodium comme le sel pentasodique de l'acide éthylènediamine tétra(méthylène phosphonique) ; les polymères notamment polyaminés tels que les polyalkylène polyamines et leurs dérivés, et en particulier la polyéthylène imine, et les dendrimères à activité chélatante ; les protéines comme la spermine, la spermidine, la transférine, la ferritine ; les acides carboxyliques comme l'acide phytique, l'acide citrique, l'acide malique, l'acide nitrilo-acétique, l'acide fumarique, l'acide tartrique, l'acide succinique, l'acide oxalique, l'acide mucique; l'acide méthylglycine diacétique, l'acide N-lauroyl-N,N',N'-tri-acétique éthylènediamine, l'acide iminodisuccinique, l'acide N,N-dicarboxyméthyl L-glutamique l'acide éthylènediamine-N,N'-dissucinique, le mésylate de desferrioxamine; le glucono delta-lactone, le gluconate de sodium, de potassium et/ou de calcium ; l'acide phosphorique, le bitartrate de potassium, l'acétate de sodium, le sorbitol et les mélanges de ces séquestrants.

Les agents séquestrant préférés sont l'acide éthylène-diamine-tétraacétique (EDTA), le glucono delta-lactone, le gluconate de sodium, de potassium et/ou de calcium ; l'acide citrique, l'acide phosphorique, l'acide tartrique, le bitartrate de potassium, l'acétate de sodium, le sorbitol.

Préférentiellement, on utilise l'EDTA.

De préférence, les teneurs d'agent séquestrant vont de 0,01% à 1%, et de préférence de 0,05 à 0,5%, en masse par rapport à la masse totale de ladite composition.

L'invention concerne tout particulièrement une composition, notamment une composition cosmétique, comprenant parmi les ingrédients de la composition (i) au moins un ou plusieurs monoglycérides comprenant une chaine alkyl, saturée ou insaturée, comprenant de 12 à 22 atomes de carbone, et de préférence, comprenant 16 ou 18 atomes de carbone, (ii) au moins un ester tartrique diacétyl de monoglycéride comprenant une chaine alkyl, saturée ou insaturée, comprenant de 12 à 22 atomes de carbone, et de préférence, comprenant 16 ou 18 atomes de carbone, (iii) au moins un pigment et/ou charge minérale enrobé, et (iv) éventuellement au moins un agent séquestrant.

Avantageusement, la composition de l'invention est une composition, notamment une composition cosmétique, comprenant parmi les ingrédients de la composition (i) au moins un ou plusieurs monoglycérides comprenant une chaine alkyl, saturée ou insaturée, comprenant de 12 à 22 atomes de carbone, (ii) au moins un ester tartrique diacétyl de monoglycéride comprenant une chaine alkyl, saturée ou insaturée, comprenant de 12 à 22 atomes de carbone, et de préférence, comprenant 16 ou 18 atomes de carbone, (iii) au moins un pigment et/ou charge minérale enrobé, et (iv) éventuellement au moins un des agents séquestrant suivants ou l'une quelconque de leurs combinaisons : l'acide éthylène-diamine-tétraacétique (EDTA), le glucono delta-lactone, le gluconate de sodium, de potassium et/ou de calcium ; l'acide citrique, l'acide phosphorique, l'acide tartrique, le bitartrate de potassium, l'acétate de sodium, le sorbitol, et de préférence l'EDTA.

Avantageusement, la composition de l'invention est une composition, notamment une composition cosmétique, comprenant parmi les ingrédients de la composition (i) au moins un ou plusieurs monoglycérides comprenant une chaine alkyl, saturée ou insaturée, comprenant 16 ou 18 atomes de carbone, (ii) au moins un ester tartrique diacétyl de monoglycéride comprenant une chaine alkyl, saturée ou insaturée, comprenant 16 ou 18 atomes de carbone, (iii) au moins un pigment et/ou charge minérale enrobé, et (iv) éventuellement au moins un agent séquestrant au moins un agent séquestrant, de préférence choisi parmi les agents suivants ou l'une quelconque de leurs combinaisons : l'acide éthylène-diamine-tétraacétique (EDTA), le glucono delta-lactone, le gluconate de sodium, de potassium et/ou de calcium ; l'acide citrique, l'acide phosphorique, l'acide tartrique, le bitartrate de potassium, l'acétate de sodium, le sorbitol, et de préférence l'EDTA.

Selon un mode de réalisation, la composition comprend une teneur en monoglycéride de 1 à 20%, une teneur en ester tartrique de monoglycéride de 0,05% à 2%, de 0,1% à 4% de pigments et/ou charges minérales enrobés, et éventuellement de 0,01% à 1% d'agent séquestrant, les pourcentages étant exprimés en masse par rapport à la masse de la composition totale.

Selon un mode de réalisation, la composition comprend une teneur en monoglycéride de 2 à 10%, une teneur en ester tartrique de monoglycéride de 0,1 à 1%, %, de préférence de 0,1% à 3% de pigments et/ou charges minérales enrobés, et éventuellement de 0,01% à 1% d'agent séquestrant, les pourcentages étant exprimés en masse par rapport à la masse de la composition totale.

Selon un mode de réalisation, la composition comprend une teneur en monoglycéride de 3 à 8 %, une teneur en ester tartrique de monoglycéride, de 0,1 à 0,5 % de pigments et/ou charges minérales enrobés, et éventuellement de 0,05 à 0,5% d'agent séquestrant, les pourcentages étant exprimés en masse par rapport à la masse de la composition totale.

L'invention couvre spécifiquement une composition comprenant comme ingrédients : MONOGLYCERIDE ; ESTER TARTRIQUE DE MONOGLYCERIDES ; OXYDE DE TITANE ENROBE DE POLYDIMETHYLSILOXANE ; et éventuellement : ACIDE ETHYLENE DIAMINE TETRACETIQUE, SEL DISODIQUE ; associés par exemple à: PHENOXYETHANOL ; XANTHAN ; 2-ACRYLOYLAMINO-2-METHYLPROPANE-1-SULFONIC ACID (AMPS) ; POLYDIMETHYLSILOXANE (PDMS) ; EAU ; GLYCERINE ; PROPYLENE GLYCOL.

### Autres ingrédients

Une certaine quantité d'huile peut être introduite dans les compositions selon l'invention.

On peut utiliser une ou plusieurs huiles couramment utilisées en cosmétique, notamment choisies parmi une huile minérale, végétale, siliconée.

De préférence le ou les huiles sont présentes dans une teneur totale allant de 0,5 à 35 % en masse par rapport à la masse de la composition totale.

Les compositions cosmétiques selon l'invention peuvent contenir les ingrédients ou additifs habituels en cosmétiques : pigments, colorants, actifs biologiques (agents anti-âge, anti-peau grasse, blanchissant la peau, anti-transpirant, anti-oxydants, etc.), filtres solaires, polymères filmogènes, charges diffusantes, huiles et corps gras, hydratants, émollients, et l'une quelconque de leurs combinaisons.

L'invention concerne encore l'utilisation d'au moins un pigment enrobé et/ou d'au moins une charge minérale enrobée, éventuellement en association avec au moins un agent séquestrant, pour améliorer la stabilité d'une composition, notamment une composition cosmétique, comprenant au moins un monoglycéride et au moins un ester tartrique diacétyl de monoglycéride.

L'invention concerne aussi une méthode de soin cosmétique comprenant l'application d'une composition telle que définie selon l'invention sur une zone de peau, notamment pour limiter la brillance ou l'aspect luisant de la peau au niveau de la zone d'application, en particulier sous un climat tropical.

La présente invention va maintenant être décrite de manière plus spécifique par le biais d'exemples.

Dans les exemples, la température est mentionnée en degré Celsius, et correspond à la température ambiante (20-25°C), sauf indication contraire, et la pression est la pression atmosphérique au niveau de la mer, sauf indication contraire. Par ailleurs, les pourcentages sont mentionnés en masse par rapport à la masse totale sauf indication contraire.

### Exemples

### Exemple 1 et 2 : Préparation de compositions cosmétiques selon la présente invention

**Tableau 1**

| Nom Chimique | EXEMPLE 1 | EXEMPLE 2 | PHASES |
|---|---|---|---|
| MONOGLYCERIDE - TEGIN 90 PELLETS EVONIK | 5 | 5 | A |
| ESTER TARTRIQUE DE MONOGLYCERIDES - PANODAN A2020 KOSHER DANISCO | 0,2* | 0,2* | |
| PHENOXYETHANOL | 0,5 | 0,5 | |
| EAU | 83 | 82,5 | |
| ACIDE ETHYLENE DIAMINE TETRACETIQUE, SEL DISODIQUE | - | 0,1 | |
| GLYCERINE | 5 | 5 | B |
| PROPYLENE GLYCOL | 2 | 2 | |
| GOMME DE XANTHANE | 0,2 | 0,2 | C |
| ACRYLAMIDE/SODIUM ACRYLOYLDIMETHYLTAURATE COPOLYMER (and) ISOHEXADECANE (and) POLYSORBATE 80 | 2 | 2 | |
| POLY DIMETHYLSILOXANE (5 cst) | 2 | 2 | |
| OXYDE DE TITANE ENROBE DE POLY DIMETHYLSILOXANE (98/2) (CI: 77891) | 0,5 | 0,5 | |

| | | | |
|---|---|---|---|
| *0,2% a 80% en masse de Matière Active (MA) | | | |

1/ Introduire le mélange de la phase A dans un fondoir, maintenir la température à 65°C pendant 45 minutes sous agitation à 250 tours/min jusqu'à obtention d'une phase homogène et opalescente.
2/ Ajouter la phase B, arrêter le flux thermique et laisser refroidir sous agitation vive entre 400 et 600 tours/min
3/ En final, ajouter la phase C comprenant le polymère et la charge sous agitation à température ambiante.

### Exemple 3 : Stabilité des compositions selon l'invention

**Tableau 2**

| Nom chimique | COMPARATIF | EXEMPLE 3 | EXEMPLE 4 |
|---|---|---|---|
| ACIDE ETHYLENE DIAMINE TETRACETIQUE, SEL DISODIQUE | - | - | 0,1 |
| OXYDE DE TITANE ENROBE DE POLY DIMETHYLSILOXANE | - | 0,5 | 0,5 |
| PHENOXYETHANOL | 0,5 | 0,5 | 0,5 |
| GOMME DE XANTHANE | 0,2 | 0,2 | 0,2 |
| ACRYLAMIDE/SODIUM ACRYLOYLDIMETHYLTAURATE COPOLYMER (and) ISOHEXADECANE (and) POLYSORBATE 80 - SIMULGEL 600 - SEPPIC | 2 | 2 | 2 |
| PDMS | 2 | 2 | 2 |
| EAU | 83,1 | 83 | 82,5 |
| GLYCERINE | 5 | 5 | 5 |
| PROPYLENE GLYCOL | 2 | 2 | 2 |
| MONOGLYCERIDE - TEGIN 90 PELLETS EVONIK | 5 | 5 | 5 |
| ESTER TARTRIQUE DE MONOGLYCERIDES - PANODAN A2020 KOSHER - DANISCO | 0,2* | 0,2* | 0,2* |

| | | | |
|---|---|---|---|
| *0,2% à 80% en masse de Matière Active (MA) | | | |

Pour évaluer la stabilité au stockage des compositions selon l'invention on les soumet à un test de vieillissement accéléré qui consiste à les stocker une semaine dans une étuve thermostatée à 55°C.

**Tableau 3**

| | COMPARITIF | EXEMPLE 1 | EXEMPLE 2 |
|---|---|---|---|
| Evolution de l'aspect macroscopique après 1 semaine à 55°C | Instable : devient nacré et opalescent | Conforme (identique au type frais) Stable : crème lisse et blanche | Conforme (identique au type frais) Stable : crème lisse et blanche |

On entend par « type frais » la formulation de l'exemple considéré après préparation de la formulation mais avant mise en œuvre du test de vieillissement accéléré.

Les compositions de l'invention sont stables de manière satisfaisante, et sont donc conformes au but recherché.

### Performance perçue

Une formule selon l'invention a été testée par des femmes à peau grasse en Inde, en Chine et au Brésil (auto-application sur 14 femmes pendant 7 jours). A la fin de la période d'essai, les femmes sont satisfaites par la performance et les propriétés cosmétiques décomposition de l'invention. L'efficacité des compositions de l'invention pour lutter contre la luisance ou la brillance de la peau au cours de la journée est perçue dans les trois pays. Le produit répond aux attentes des individus présentant une peau grasse, notamment sous un climat tropical.

Les femmes ayant appliqué sur leur peau les compositions selon la présente invention considèrent qu'elles sont confortables, c'est-à-dire que l'application ne présente pas de gêne particulière mais au contraire procure une sensation de confort et un effet d'hydratation de la peau.

Les compositions de l'invention répondent donc aux problématiques fixées initialement.

Une formule non-conforme à l'invention a été testée et ne permet pas d'obtenir un résultat satisfaisant en termes de matité.

## Revendications

1. Composition, notamment composition cosmétique, **caractérisée en ce qu'**elle comprend une phase aqueuse, une phase lipogel, au moins un monoglycéride et au moins un ester tartrique de monoglycéride, et au moins un pigment enrobé et/ou au moins une charge minérale enrobée.

2. Composition, selon la revendication 1, **caractérisée en ce qu'**elle comprend plus de 50% en masse de phase aqueuse par rapport à la masse totale de la composition.

3. Composition, selon la revendication 1 ou 2, **caractérisée en ce qu'**elle comprend à titre de monoglycéride un ou plusieurs monoglycérides comprenant une chaine alkyl, saturée ou insaturée, comprenant de 12 à 22 atomes de carbone.

4. Composition, selon l'une quelconque des revendications 1 à 3, **caractérisée en ce que** le monoglycéride comprend une chaine alkyl, saturée ou insaturée, comprenant 16 ou 18 atomes de carbone.

5. Composition, selon l'une quelconque des revendications 1 à 4, **caractérisée en ce que** la composition comprend une teneur en monoglycéride de 1 à 20%, préférentiellement de 2 à 10% et très préférentiellement de 3 à 8 % en masse par rapport à la masse de la composition totale.

6. Composition, selon l'une quelconque des revendications 1 à 5, **caractérisée en ce que** l'ester tartrique de monoglycéride est un ester tartrique diacétyl de monoglycéride comprenant une chaine alkyl, saturée ou insaturée, comprenant de 12 à 22 atomes de carbone, et de préférence, comprenant 16 ou 18 atomes de carbone.

7. Composition, selon l'une quelconque des revendications 1 à 6, **caractérisée en ce que** la teneur en ester tartrique de monoglycéride va de 0,05% à 5 %, préférentiellement de 0,1 à 2 %, et très préférentiellement de 0,1 à 1% en masse par rapport à la masse de la composition totale.

8. Composition, selon l'une quelconque des revendications 1 à 7, **caractérisée en ce qu'**elle comprend une teneur en pigments et/ou charges minérales enrobés de 0,1% à 4%, de préférence de 0,2% à 3%, en masse par rapport à la masse totale de ladite composition.

9. Composition, selon l'une quelconque des revendications 1 à 8, **caractérisée en ce que** l'agent de traitement représente de 0,1 à 50% en masse, et en particulier de 0,5 à 5% en masse, de la masse totale du pigment enrobé ou de la charge minérale enrobée.

10. Composition, selon l'une quelconque des revendications 1 à 9, **caractérisée en ce qu'**elle comprend au moins un agent séquestrant.

11. Composition, selon l'une quelconque des revendications 1 à 10, **caractérisée en ce qu'**elle comprend au moins un agent séquestrant parmi les suivants ou l'une quelconque de leurs combinaisons : l'acide éthylène-diamine-tétraacétique (EDTA), le glucono delta-lactone, le gluconate de sodium, de potassium et/ou de calcium ; l'acide citrique, l'acide phosphorique, l'acide tartrique, le bitartrate de potassium, l'acétate de sodium, le sorbitol, et de préférence l'EDTA.

12. Composition, selon l'une quelconque des revendications 1 à 11, **caractérisée en ce qu'**elle comprend une teneur d'agent séquestrant de 0,01 % à 1%, et de préférence de 0,05 à 0,5%, en masse par rapport à la masse totale de ladite composition.

13. Utilisation d'au moins un pigment enrobé et/ou d'au moins une charge minérale enrobée, éventuellement en association avec au moins un agent séquestrant, pour améliorer la stabilité d'une composition, notamment une composition cosmétique, comprenant au moins un monoglycéride et au moins un ester tartrique diacétyl de monoglycéride.

14. Méthode de soin cosmétique, **caractérisée en ce qu'**elle comprend l'application d'une composition telle que définie selon l'une quelconque des revendications 1 à 12 sur une zone de peau, notamment pour limiter la brillance ou l'aspect luisant de la peau au niveau de la zone d'application, en particulier sous un climat tropical.

## Patentansprüche

1. Zusammensetzung, insbesondere kosmetische Zusammensetzung, **dadurch gekennzeichnet, dass** sie eine wässrige Phase, eine Lipogelphase, mindestens ein Monoglycerid und mindestens einen Weinsäureester eines Monoglycerids und mindestens ein beschichtetes Pigment und/oder mindestens einen beschichteten mineralischen Füllstoff umfasst.

2. Zusammensetzung nach Anspruch 1, **dadurch gekennzeichnet, dass** sie mehr als 50 Massenprozent wässrige Phase mit Bezug auf die Gesamtmasse der Zusammensetzung umfasst.

3. Zusammensetzung nach Anspruch 1 oder 2, **dadurch gekennzeichnet, dass** sie als Monoglycerid ein oder mehrere Monoglyceride umfasst, umfassend eine Alkylkette, gesättigt oder ungesättigt, umfassend von 12 bis 22 Kohlenstoffatome.

4. Zusammensetzung nach einem der Ansprüche 1 bis 3, **dadurch gekennzeichnet, dass** das Monoglycerid eine Alkylkette, gesättigt oder ungesättigt, umfasst, umfassend von 16 oder 18 Kohlenstoffatome.

5. Zusammensetzung nach einem der Ansprüche 1 bis 4, **dadurch gekennzeichnet, dass** die Zusammensetzung einen Monoglyceridgehalt von 1 bis 20, vorzugsweise von 2 bis 10 und ganz bevorzugt von 3 bis 8 Massenprozent mit Bezug auf die Masse der Gesamtzusammensetzung umfasst.

6. Zusammensetzung nach einem der Ansprüche 1 bis 5, **dadurch gekennzeichnet, dass** der Weinsäureester eines Monoglycerids ein Diacetylweinsäureester eines Monoglycerids ist, umfassend eine Alkylkette, gesättigt oder ungesättigt, umfassend von 12 bis 22 Kohlenstoffatome und vorzugsweise umfassend 16 oder 18 Kohlenstoffatome.

7. Zusammensetzung nach einem der Ansprüche 1 bis 6, **dadurch gekennzeichnet, dass** der Gehalt an Weinsäureester eines Monoglycerids von 0,05 bis 5, vorzugsweise von 0,1 bis 2 und ganz bevorzugt von 0,1 bis 1 Massenprozent mit Bezug auf die Masse der Gesamtzusammensetzung reicht.

8. Zusammensetzung nach einem der Ansprüche 1 bis 7, **dadurch gekennzeichnet, dass** sie einen Gehalt an Pigmenten und/oder beschichteten mineralischen Füllstoffen von 0,1 bis 4, vorzugsweise von 0,2 bis 3 Massenprozent mit Bezug auf die Gesamtmasse der Zusammensetzung umfasst.

9. Zusammensetzung nach einem der Ansprüche 1 bis 8, **dadurch gekennzeichnet, dass** das Behandlungsmittel von 0,1 bis 50 Massenprozent und insbesondere von 0,5 bis 5 Massenprozent der Gesamtmasse des beschichteten Pigments oder des beschichteten mineralischen Füllstoffs umfasst.

10. Zusammensetzung nach einem der Ansprüche 1 bis 9, **dadurch gekennzeichnet, dass** sie mindestens ein Maskierungsmittel umfasst.

11. Zusammensetzung nach einem der Ansprüche 1 bis 10, **dadurch gekennzeichnet, dass** sie mindestens ein Maskierungsmittel aus den Folgenden oder einer ihrer Kombinationen umfasst: Ethylendiamintetraessigsäure (EDTA), Glucono-delta-Lacton, Natrium-, Kalium- und/oder Kalziumgluconat, Zitronensäure, Phosphorsäure, Weinsäure, Kaliumbitartrat, Natriumacetat, Sorbitol und vorzugsweise EDTA.

12. Zusammensetzung nach einem der Ansprüche 1 bis 11, **dadurch gekennzeichnet, dass** sie einen Gehalt an Maskierungsmittel von 0,01 bis 1, vorzugsweise von 0,05 bis 0,5 Massenprozent mit Bezug auf die Gesamtmasse der Zusammensetzung umfasst.

13. Verwendung mindestens eines beschichteten Pigments und/oder mindestens eines beschichteten mineralischen Füllstoffs, eventuell in Verbindung mit mindestens einem Maskierungsmittel, um die Stabilität einer Zusammensetzung zu verbessern, insbesondere einer kosmetischen Zusammensetzung, umfassend mindestens einen Diacetylweinsäureester eines Monoglycerids.

14. Verfahren zur kosmetischen Pflege, **dadurch gekennzeichnet, dass** es die Anwendung einer Zusammensetzung, wie nach einem der Ansprüche 1 bis 12 definiert, auf eine Zone der Haut umfasst, insbesondere, um den Glanz oder den leuchtenden Aspekt der Haut auf der Ebene der Anwendungszone, insbesondere unter einem tropischen Klima, zu begrenzen.

## Claims

1. Composition, especially a cosmetic composition, **characterized in that** it comprises an aqueous phase, a lipogel phase, at least one monoglyceride and at least one tartaric ester of monoglyceride, and at least one coated pigment and/or at least one coated mineral filler.

2. Composition, according to claim 1, **characterized in that** it comprises more than 50% in mass of aqueous phase relative to the total mass of the composition.

3. Composition, according to claim 1 or 2, **characterized in that** it comprises as a monoglyceride one or more monoglycerides comprising an alkyl chain, saturated or unsaturated, comprising from 12 to 22 carbon atoms.

4. Composition, according to any one of claims 1 to 3, **characterized in that** the monoglyceride comprises an alkyl chain, saturated or unsaturated, comprising 16 or 18 carbon atoms.

5. Composition, according to any one of claims 1 to 4, **characterized in that** the composition comprises a monoglyceride content from 1% to 20%, preferably from 2 to 10% and very preferably from 3 to 8% by mass based on the total mass of said composition.

6. Composition, according to any one of claims 1 to 5, **characterized in that** the tartaric ester of monoglyceride is a diacetyl tartaric ester of monoglyceride comprising an alkyl chain, saturated or unsaturated, comprising from 12 to 22 carbon atoms, and preferably comprising 16 or 18 carbon atoms.

7. Composition, according to any one of claims 1 to 6, **characterized in that** the tartaric ester of monoglyceride content ranges from 0.05% to 5 %, preferably from 0.1 to 2 % and very preferably from 0.1 to 1% by mass based on the total mass of said composition.

8. Composition, according to any one of claims 1 to 7, **characterized in that** it comprises a coated pigment and/or coated mineral filler content from 0.1% to 4% and preferably from 0.2 to 3% by mass based on the total mass of said composition.

9. Composition according to any one of claims 1 to 8, **characterized in that** the treatment agent represents from 0.1 to 50% by mass, and particularly from 0.5 to 5 % by mass, of the total mass of the coated pigment or the coated mineral filler.

10. Composition according to any of claims 1 to 9, **characterized in that** it comprises a least one sequestering agent.

11. Composition according to any of claims 1 to 10, **characterized in that** it comprises a least one sequestering agent from among the following or any combination thereof: ethylene diamine tetraacetic acid (EDTA), glucono delta-lactone, sodium gluconate, potassium and/or calcium; citric acid, phosphoric acid, tartaric acid, potassium bitartrate, sodium acetate, sorbitol, and preferably EDTA.

12. Composition, according to any one of claims 1 to 11, **characterized in that** it comprises a sequestering agent content from 0.01% to 1% and preferably from 0.05 to 0.5% by mass based on the total mass of said composition.

13. Use of at least one coated pigment and/or at least one coated mineral filler, optionally in combination with at least one sequestering agent, for improving the stability of a composition, especially a cosmetic composition, comprising at least one monoglyceride and at least one diacetyl tartaric ester of monoglyceride.

14. Cosmetic treatment method, **characterized in that** it comprises applying a composition such as defined according to any of claims 1 to 12 on an area of skin, in particular to limit the gloss or shiny appearance of the skin at the area of application, especially in a tropical climate.
